# EUROPÄISCHE PATENTANMELDUNG

(11) **EP 0 785 201 A1**
(43) Veröffentlichungstag der Anmeldung: **23.07.1997**
(21) Anmeldenummer: 97100114.4
(22) Anmeldetag: 07.01.1997
(51) Int. Cl.: C07D 513/04, A61K 31/435, C07D 498/04, C07D 471/04

(54) **Heteroatomhaltige Cyclopentanopyridyl-Oxazolidinone**

(30) Priorität: 18.01.1996 DE 19601627
(71) Anmelder: BAYER AG, 51368 Leverkusen (DE)
(72) Erfinder: Stolle, Andreas, Dr., 42115 Wuppertal (DE); Häbich, Dieter, Dr., 42115 Wuppertal (DE); Riedl, Bernd, Dr., 42329 Wuppertal (DE); Ruppelt, Martin, Dr., 42115 Wuppertal (DE); Bartel, Stephan, Dr., 51465 Bergisch Gladbach (DE); Guarnieri, Walter, Dr., 53909 Zülpich (DE); Wild, Hanno, Dr., Orange, CT 06477 (DE); Endermann, Rainer, Dr., 42113 Wuppertal (DE); Kroll, Hein-Peter, Dr., 42115 Wuppertal (DE)

(57) **Zusammenfassung**

Die vorliegende Erfindung betrifft heteroatomhaltige Cyclopentanopyridyl-Oxazolidinone, Verfahren zu ihrer Herstellung und ihre Verwendung als Arzneimittel, insbesondere als antibakterielle Arzneimittel.

## Beschreibung

Die vorliegende Erfindung betrifft heteroatomhaltige Cyclopentanopyridyl-Oxazolidinone, Verfahren zu ihrer Herstellung und ihre Verwendung als Arzneimittel, insbesondere als antibakterielle Arzneimittel.

Aus EP 609 905 sind 3-(Stickstoff-substituierte)phenyl-5-beta-amidomethyloxazolidin-2-one mit antibakterieller Wirkung bekannt.

Ferner sind in der WO 93 08 179 A und EP 657 440 Oxazolidinonderivate mit einer Monoaminoxidase inhibitorischen Wirkung und in der EP 645 376 mit Wirkung als Adhäsionsrezeptor-Antagonisten publiziert.

Die vorliegende Erfindung betrifft heteroatomhaltige Cyclopentanopyridyl-Oxazolidinone der allgemeinen Formel (I) 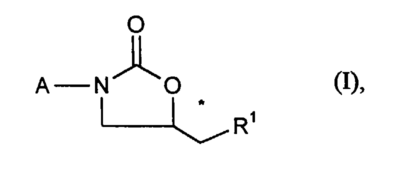 in welcher
- A: für einen Rest der Formel 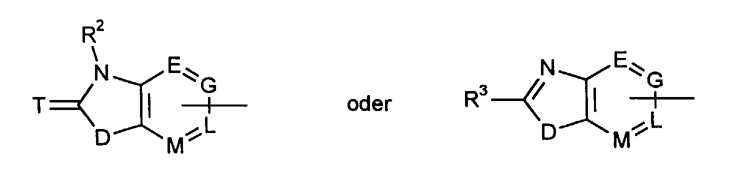 steht,
worin
- E, G, L und M: gleich oder verschieden sind und mindestens einer dieser Substituenten ein Stickstoffatom bedeutet und die anderen den Rest der Formel -CR⁴ bedeuten,
worin
- R⁴: Wasserstoff, Methyl oder Halogen bedeutet,
- R²: Wasserstoff, Cycloalkyl oder Cycloalkylcarbonyl mit jeweils 3 bis 8 Kohlenstoffatomen bedeutet, oder geradkettiges oder verzweigtes Alkyl mit bis zu 8 Kohlenstoffatomen bedeutet, das gegebenenfalls durch Hydroxy, Halogen, durch geradkettiges oder verzweigtes Alkoxy, Alkoxycarbonyl oder Alkylthio mit jeweils bis zu 6 Kohlenstoffatomen oder durch einen Rest der Formel -NR⁶R⁷ substituiert ist,
worin
- R⁶ und R⁷: gleich oder verschieden sind und
Wasserstoff, Cycloalkyl, Phenyl oder geradkettiges oder verzweigtes Alkyl mit bis zu 4 Kohlenstoffatomen bedeuten,
- R³: geradkettiges oder verzweigtes Alkyl oder Thioalkyl mit jeweils bis zu 8 Kohlenstoffatomen bedeutet,
- D: ein Sauerstoff- oder Schwefelatom oder eine Gruppe der Formel -NR⁵ bedeutet,
worin
- R⁵: die oben angegebene Bedeutung von R² hat und mit dieser gleich oder verschieden sind,
- T: bedeutet ein Sauerstoff- oder Schwefelatom,
- R¹: für Azido, Hydroxy oder für eine Gruppe der Formel -OR⁸, O-SO₂R⁹ oder -NR¹⁰R¹¹ steht,
worin
- R⁸: geradkettiges oder verzweigtes Acyl mit bis zu 8 Kohlenstoffatomen oder eine Hydroxyschutzgruppe bedeutet,
- R⁹: geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
- R¹⁰ und R¹¹: gleich oder verschieden sind und Cycloalkyl mit 3 bis 6 Kohlenstoffatomen, Wasserstoff, Phenyl oder geradkettiges oder verzweigtes Alkyl oder Alkoxy mit jeweils bis zu 8 Kohlenstoffatomen oder eine Aminoschutzgruppe bedeuten,
oder
- R¹⁰ oder R¹¹: eine Gruppe der Formel -CO-R¹², -CS-R^{12'}, P(O)(OR¹³)(OR¹⁴) oder -SO₂-R¹⁵ bedeutet,
worin
- R¹² und R^{12'}: gleich oder verschieden sind und
Wasserstoff, Cycloalkyl mit 3 bis 6 Kohlenstoffatomen, Trifluormethyl, geradkettiges oder verzweigtes Alkoxy mit bis zu 8 Kohlenstoffatomen, Phenyl, Benzyloxy oder Wasserstoff bedeuten, oder
geradkettiges oder verzweigtes Alkyl mit bis zu 8 Kohlenstoffatomen bedeuten, das gegebenenfalls durch Cyano, Halogen oder Trifluormethyl substituiert ist,
oder
geradkettiges oder verzweigtes Thioalkyl oder Acyl mit jeweils bis zu 6 Kohlenstoffatomen bedeuten,
oder
eine Gruppe der Formel -NR¹⁶R¹⁷ bedeuten,
worin
- R¹⁶ und R¹⁷: gleich oder verschieden sind und Wasserstoff, Phenyl oder geradkettiges oder verzweigtes Alkyl mit bis zu 6 Kohlenstoffatomen bedeuten,
oder
einen 5-gliedrigen aromatischen Heterocyclus mit bis zu 3 Heteroatomen aus der Reihe S, N und/oder O bedeuten,
- R¹³ und R¹⁴: gleich oder verschieden sind und Wasserstoff oder geradkettiges oder verzweigtes Alkyl mit bis zu 4 Kohlenstoffatomen bedeuten,
- R¹⁵: geradkettiges oder verzweigtes Alkyl mit bis zu 4 Kohlenstoffatomen oder Phenyl bedeutet
und deren Salze.

Physiologisch unbedenkliche Salze der heteroatomhaltigen Cyclopentanopyridyl-Oxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Als Salze können außerdem Reaktionsprodukte mit C₁-C₄-Alkylhalogeniden, insbesondere C₁-C₄-Alkyljodiden fungieren.

Heteroyclus steht im allgemeinen für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl oder Tetrahydropyranyl.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren und deren jeweilige Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- A: für einen Rest der Formel 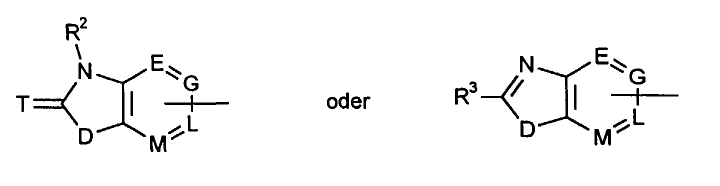 steht,
worin
- E, G, L und M: gleich oder verschieden sind und mindestens einer der Substituenten ein Stickstoffatom bedeutet und die anderen den Rest der Formel -CR⁴ bedeuten,
worin
- R⁴: Wasserstoff, Fluor, Chlor oder Brom bedeutet,
- R²: Wasserstoff, Cyclopropyl, Cyclobutyl, Cyclopentyl, Cyclohexyl oder Cycloheptyl bedeutet, oder
geradkettiges oder verzweigtes Alkyl mit bis zu 6 Kohlenstoffatomen bedeutet, das gegebenenfalls durch Hydroxy, Fluor, Chlor, Brom, durch geradkettiges oder verzweigtes Alkoxy, Alkoxycarbonyl oder Alkylthio mit jeweils bis zu 4 Kohlenstoffatomen oder durch einen Rest der Formel -NR⁶R⁷ substituiert ist,
worin
- R⁶ und R⁷: gleich oder verschieden sind und
Wasserstoff, Cyclopropyl, Cyclobutyl, Cyclopentyl, Cyclohexyl, Phenyl oder geradkettiges oder verzweigtes Alkyl mit bis zu 3 Kohlenstoffatomen bedeuten,
- R³: geradkettiges oder verzweigtes Alkyl oder Thioalkyl mit jeweils bis zu 6 Kohlenstoffatomen bedeutet,
- D: ein Sauerstoff- oder Schwefelatom oder eine Gruppe der Formel -NR⁵ bedeutet,
worin
- R⁵: die oben angegebene Bedeutung von R² hat und mit dieser gleich oder verschieden ist,
- T: ein Sauerstoff- oder Schwefelatom bedeutet,
- R¹: für Azido, Hydroxy oder für eine Gruppe der Formel -OR⁸, O-SO₂R⁹ oder -NR¹⁰R¹¹ steht,
worin
- R⁸: geradkettiges oder verzweigtes Acyl mit bis zu 6 Kohlenstoffatomen oder Benzyl bedeutet,
- R⁹: geradkettiges oder verzweigtes Alkyl mit bis zu 3 Kohlenstoffatomen, Phenyl oder Tolyl bedeutet,
- R¹⁰ und R¹¹: gleich oder verschieden sind und
Cyclopropyl, Cyclopentyl, Cyclohexyl, Wasserstoff, Phenyl oder geradkettiges oder verzweigtes Alkyl oder Alkoxy mit jeweils bis zu 6 Kohlenstoffatomen, tert.Butoxycarbonyl oder Benzyloxycarbonyl bedeuten,
oder
- R¹⁰ oder R¹¹: eine Gruppe der Formel -CO-R¹², -CS-R^{12'}, P(O)(OR¹³)(OR¹⁴) oder -SO₂-R¹⁵ bedeutet,
worin
- R¹² und R^{12'}: gleich oder verschieden sind und
Wasserstoff, Cyclopropyl, Cyclobutyl, Cyclopentyl, Cyclohexyl, Trifluormethyl oder geradkettiges oder verzweigtes Alkoxy mit bis zu 6 Kohlenstoffatomen, Phenyl, Benzyloxy oder Wasserstoff bedeuten, oder
geradkettiges oder verzweigtes Alkyl mit bis zu 6 Kohlenstoffatomen bedeuten, das gegebenenfalls durch Cyano, Fluor, Chlor, Brom oder Trifluormethyl substituiert ist, oder
geradkettiges oder verzweigtes Thioalkyl oder Acyl mit jeweils bis zu 5 Kohlenstoffatomen bedeuten, oder
eine Gruppe der Formel -NR¹⁶R¹⁷ bedeuten,
worin
- R¹⁶ und R¹⁷: gleich oder verschieden sind und Wasserstoff, Phenyl oder geradkettiges oder verzweigtes Alkyl mit bis zu 4 Kohlenstoffatomen bedeuten, oder
Isoxazolyl, Furyl, Thienyl, Pyrryl, Oxazolyl oder Imidazolyl bedeuten,
- R¹³ und R¹⁴: gleich oder verschieden sind und Wasserstoff oder geradkettiges oder verzweigtes Alkyl mit bis zu 3 Kohlenstoffatomen bedeuten,
- R¹⁵: geradkettiges oder verzweigtes Alkyl mit bis zu 3 Kohlenstoffatomen oder Phenyl bedeutet,
und deren Salze.

Besonders bevorzugt sind Verbindungen der allgemeinen Formel (I),
in welcher
- A: für einen Rest der Formel 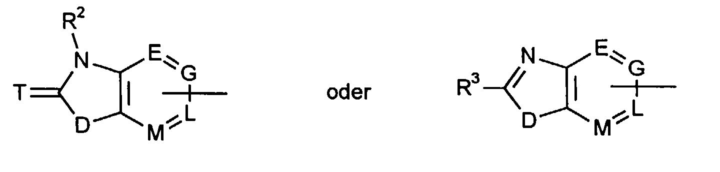 steht,
worin
- E, G, L und M: gleich oder verschieden sind und mindestens einer der Substituenten ein Stickstoffatom bedeutet und die anderen den Rest der Formel -CR⁴ bedeuten,
worin
- R⁴: Wasserstoff oder Fluor bedeutet,
- R²: Wasserstoff, Cyclopropyl, Cyclobutyl, Cyclopentyl oder Cyclohexyl bedeutet, oder
geradkettiges oder verzweigtes Alkyl mit bis zu 5 Kohlenstoffatomen bedeutet, das gegebenenfalls durch Hydroxy, Fluor, Chlor, Brom, durch geradkettiges oder verzweigtes Alkoxy, Alkoxycarbonyl oder Alkylthio mit jeweils bis zu 3 Kohlenstoffatomen oder durch einen Rest der Formel -NR⁶R⁷ substituiert ist,
worin
- R⁶ und R⁷: gleich oder verschieden sind und
Wasserstoff, Cyclopropyl, Cyclopentyl, Cyclohexyl, Phenyl oder geradkettiges oder verzweigtes Alkyl mit bis zu 3 Kohlenstoffatomen bedeuten,
- R³: geradkettiges oder verzweigtes Alkyl oder Thioalkyl mit jeweils bis zu 5 Kohlenstoffatomen bedeutet,
- D: ein Sauerstoff- oder Schwefelatom bedeutet,
- T: ein Sauerstoffatom bedeutet,
- R¹: für Azido, Hydroxy oder für eine Gruppe der Formel -OR⁸, O-SO₂R⁹ oder -NR¹⁰R¹¹ steht,
worin
- R⁸: geradkettiges oder verzweigtes Acyl mit bis zu 5 Kohlenstoffatomen oder Benzyl bedeutet,
- R⁹: Methyl, Ethyl, Phenyl oder Toluolyl bedeutet,
- R¹⁰ und R¹¹: gleich oder verschieden sind und
Cyclopropyl, Cyclopentyl, Cyclohexyl, Wasserstoff, Phenyl oder geradkettiges oder verzweigtes Alkyl oder Alkoxy mit jeweils bis zu 5 Kohlenstoffatomen, tert.Butoxycarbonyl oder Benzyloxycarbonyl bedeuten,
oder
- R¹⁰ oder R¹¹: eine Gruppe der Formel -CO-R¹², -CS-R^{12'}, P(O)(OR¹³)(OR¹⁴) oder -SO₂R¹⁵ bedeutet,
worin
- R¹² und R^{12'}: gleich oder verschieden sind und
Wasserstoff, Cyclopropyl, Cyclobutyl, Cyclopentyl, Trifluormethyl oder geradkettiges oder verzweigtes Alkoxy mit bis zu 5 Kohlenstoffatomen, Phenyl, Benzyloxy oder Wasserstoff bedeuten,
geradkettiges oder verzweigtes Alkyl mit bis zu 5 Kohlenstoffatomen bedeuten, das gegebenenfalls durch Cyano, Fluor, Chlor, Brom oder Trifluormethyl substituiert ist, oder
geradkettiges oder verzweigtes Thioalkyl- oder Acyl mit jeweils bis zu 4 Kohlenstoffatomen bedeuten, oder
eine Gruppe der Formel -NR¹⁶R¹⁷ bedeuten,
worin
- R¹⁶ und R¹⁷: gleich oder verschieden sind und Wasserstoff, Phenyl oder geradkettiges oder verzweigtes Alkyl mit bis zu 3 Kohlenstoffatomen bedeuten, oder
Isoxazolyl, Furyl, Oxazolyl oder Imidazolyl bedeuten,
- R¹³ und R¹⁴: gleich oder verschieden sind und Wasserstoff, Methyl oder Ethyl bedeuten,
- R¹⁵: Methyl oder Phenyl bedeutet
und deren Salze.

Ganz besonders bevorzugt sind Verbindungen der allgemeinen Formel (I),
in welcher
der Oxazolidinonrest in den Positionen 5 oder 6 an den stickstoffhaltigen Ring angebunden ist.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formel (II) oder (III)

   A-N=C=O (II)

   oder A-CO-N₃ (III)

   in welchen
   - A: die oben angegebene Bedeutungen hat,
   mit Lithiumbromid/(C₄H₉)₃P(O) und Epoxiden der allgemeinen Formel (IV) 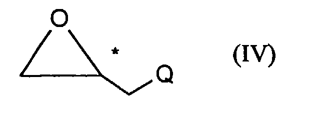 in welcher
   - Q: für C₁-C₆-Acyloxy steht,
   in inerten Lösemitteln, gegebenenfalls in Anwesenheit einer Base
   umsetzt,
   und im Fall R¹ = OH durch eine typische Esterverseifung oder durch eine typische Umesterung die Hydroxyfunktion freisetzt,
   oder
[B] Verbindungen der allgemeinen Formel (V)

   A-NH-CO₂-X (V)

   in welcher
   - A: die oben angegebene Bedeutung hat
   und
   - X: für eine typische Schutzgruppe, vorzugsweise Benzyl steht,
   in inerten Lösemitteln und in Anwesenheit einer Base, beispielsweise Lithiumalkylen oder Lithium-N-alkyl- oder Lithium-N-silylalkylamiden, vorzugsweise n-Butyllithium, mit Epoxiden der allgemeinen Formel (IV) umsetzt,
   oder
[C] im Fall R¹ = OH, zunächst Verbindungen der allgemeinen Formel (III) durch Abspaltung von Stickstoff in Alkoholen in die Verbindungen der allgemeinen Formel (Va)

   A-NH-CO₂-Y (Va)

   in welcher
   - A: die oben angegebene Bedeutung hat
   und
   - Y: für geradkettiges oder verzweigtes C₁-C₆-Alkyl, vorzugsweise n-Butyl steht,
   überführt,
   und in einem zweiten Schritt wie unter [A] beschrieben in inerten Lösemitteln und in Anwesenheit einer Base, vorzugsweise Lithium-N-alkyl- oder N-Silylalkylamiden oder n-Butyllithium, mit Epoxiden der allgemeinen Formel (IV) umsetzt,
   oder
[D] Verbindungen der allgemeinen Formel (V) zunächst durch Umsetzung mit Allylbromid in inerten Lösemitteln und in Anwesenheit einer Base in die Verbindungen der allgemeinen Formel (VI) 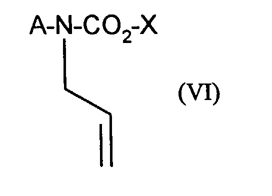 in welcher
   - A und X: die oben angegebene Bedeutung haben,
   überführt, anschließend mit Osmiumtetroxid / N-Methylmorpholin-N-oxid die Verbindungen der allgemeinen Formel (VII) 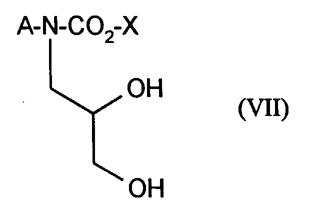 in welcher
   - A und X: die oben angegebene Bedeutung haben,
   herstellt und in einem letzten Schritt mit Basen in Acetonitril, vorzugsweise mit Kaliumcarbonat, eine Cyclisierung durchführt,
   oder
[E] Verbindungen der allgemeinen Formel (VIII) 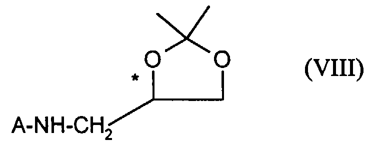 in welcher
   - A: die oben angegebene Bedeutung hat,
   entweder direkt mit Säuren und Kohlensäurediethylester
   umsetzt,
   oder zunächst durch Umsetzung der Verbindungen der allgemeinen Formel (VIII) mit Säuren die Verbindungen der allgemeinen Formel (IX) 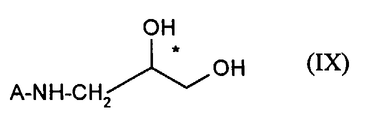 in welcher
   - A: die oben angegebene Bedeutung hat,
   herstellt,
   und anschließend in Anwesenheit eines Hilfsmittels und/oder einer Säure in inerten Lösemitteln cyclisiert,
   oder
[F] zunächst die heterocyclischen Amine (A-NH₂) mit einer Verbindung der allgemeinen Formel (IV) zu einer Verbindung der allgemeinen Formel (IX) umsetzt und anschließend mit Carbonyldiimidazol / Methylenchlorid, (Et₂O)₂CO oder Phosgen, Diphosgen oder Triphosgen wie unter [E] beschrieben cyclisiert,
   oder
[G] zunächst Verbindungen der allgemeinen Formel (Ia) 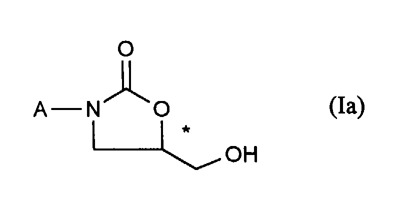 in welcher
   - A: die oben angegebene Bedeutung hat,
   durch Umsetzung mit (C₁-C₄)-Alkyl- oder Phenylsulfonsäurechloriden in inerten Lösemitteln und in Anwesenheit einer Base in die entsprechenden Verbindungen der allgemeinen Formel (Ib) 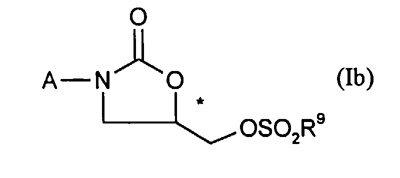 in welcher
   - A und R⁹: die oben angegebene Bedeutung haben,
   überführt,
   anschließend mit Natriumazid in inerten Lösemitteln die Azide der allgemeinen Formel (Ic) 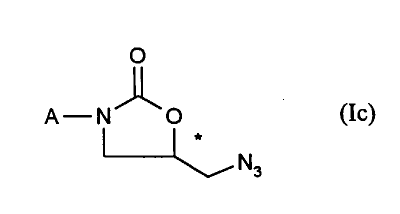 in welcher
   - A: die oben angegebene Bedeutung hat,
   herstellt,
   in einem weiteren Schritt durch Umsetzung mit (C₁-C₄-O)₃-P oder PPh₃, vorzugsweise (CH₃O)₃P in inerten Lösemitteln und mit Säuren in die Amine der allgemeinen Formel (Id) 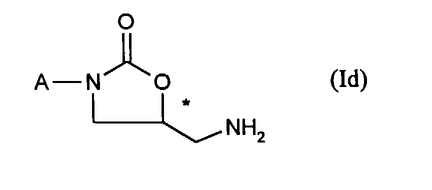 in welcher
   - A: die oben angegebene Bedeutung hat,
   überführt,
   und durch Umsetzung mit Acetanhydrid oder anderen Acylierungsmitteln der allgemeinen Formel (X)

   R¹⁸-CO-R¹² (X)

   in welcher
   - R¹²: die oben angegebene Bedeutung hat
   und
   - R¹⁸: für Halogen, vorzugsweise für Chlor oder für den Rest -OCOR¹² steht,
   in inerten Lösemitteln die Verbindungen der allgemeinen Formel (Ie) 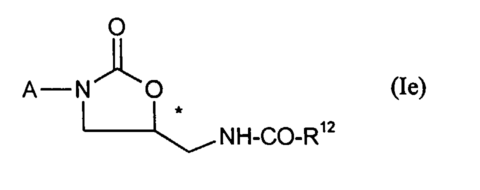 in welcher
   - A und R¹²: die oben angegebene Bedeutung haben,
   herstellt,
   und im Fall R¹ = NR¹⁰-CSR^{12'} Verbindungen der allgemeinen Formel (Id) mit Ethyldithiocarboxylaten und Triethylamin und im Fall R¹ = NR¹²-CS-NR¹⁶R¹⁷ mit Thioisocyanaten umsetzt.

Die erfindungsgemäßen Verfahren können durch folgende Formelschemata beispielhaft erläutert werden:

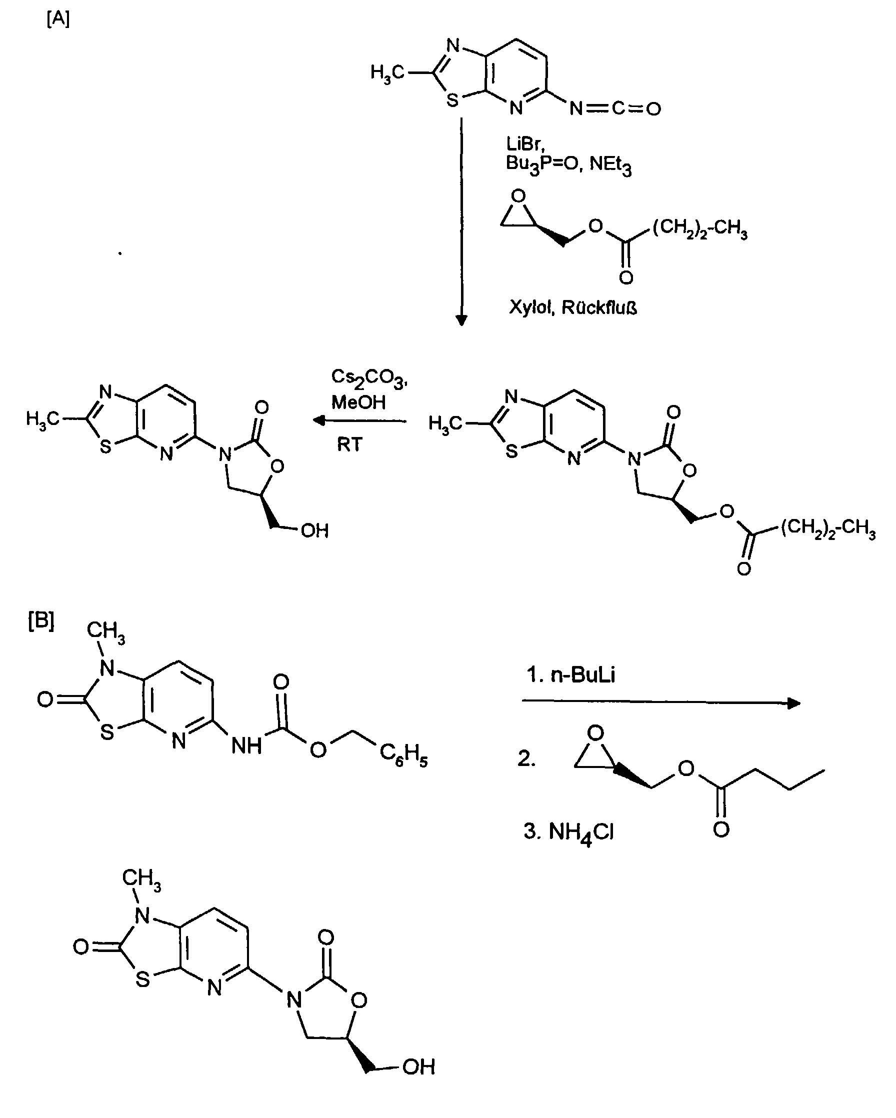

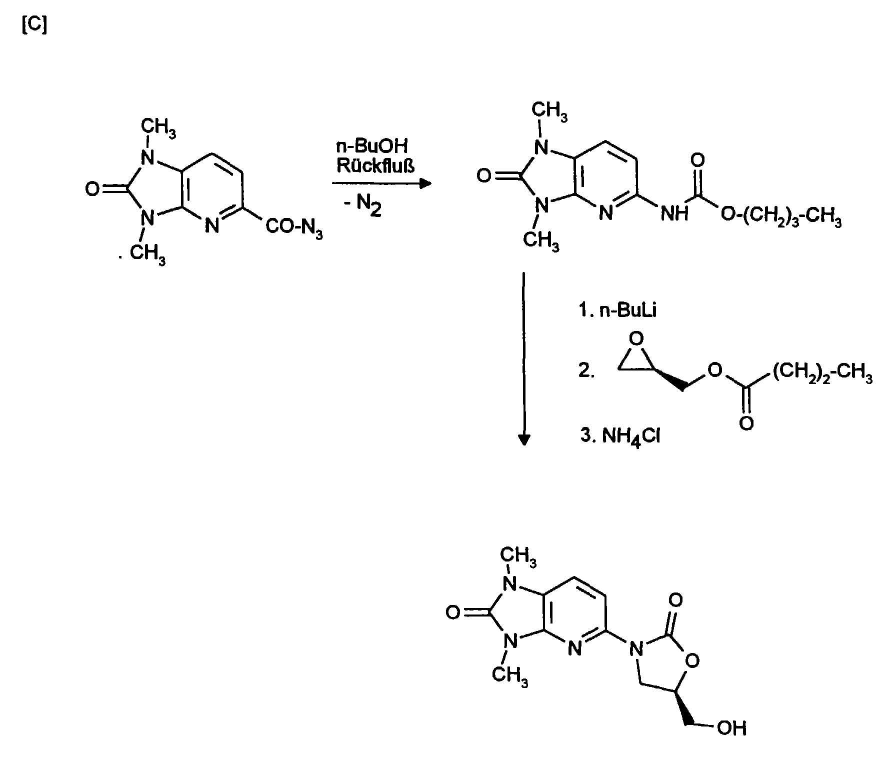

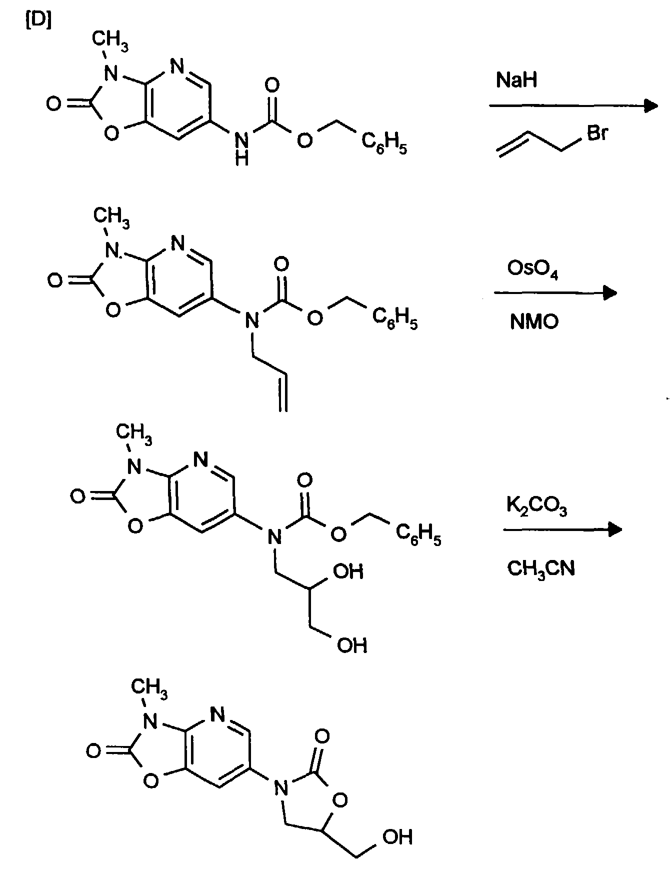

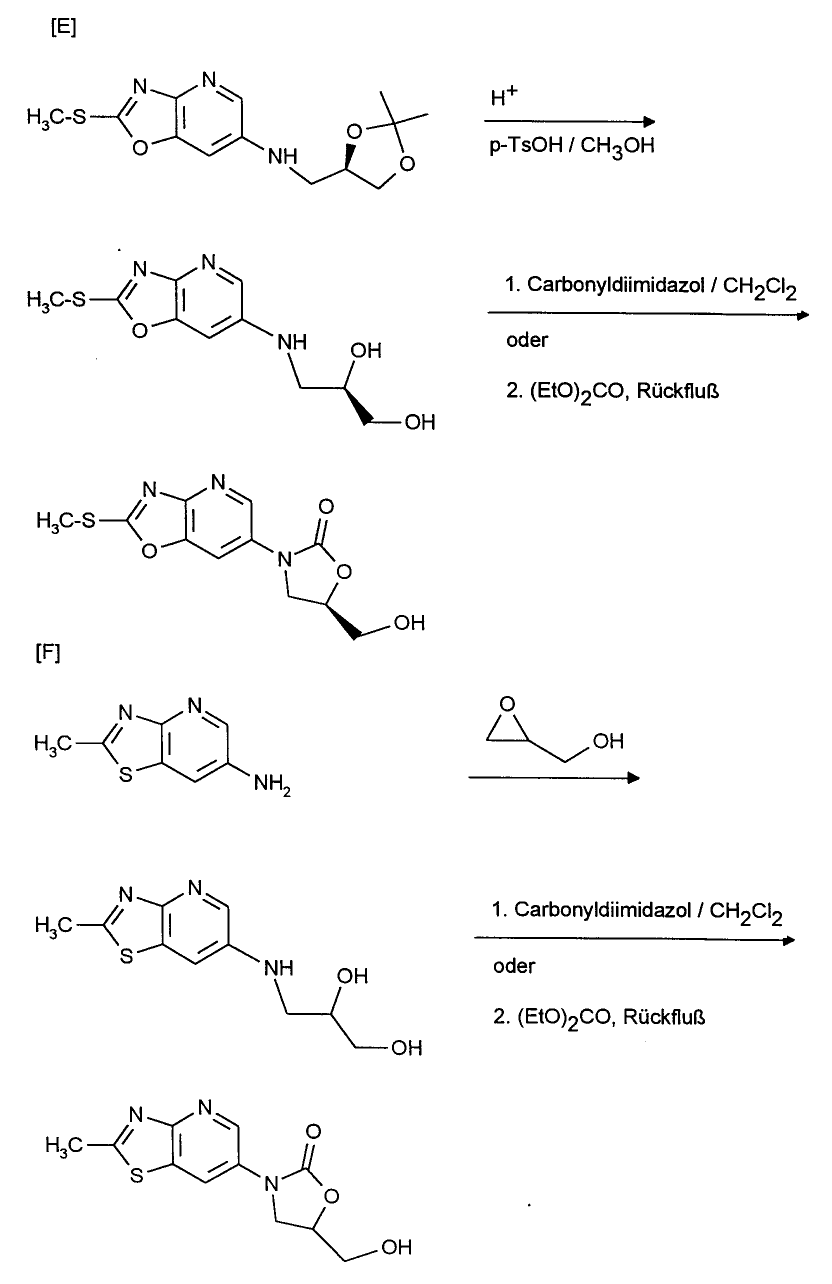

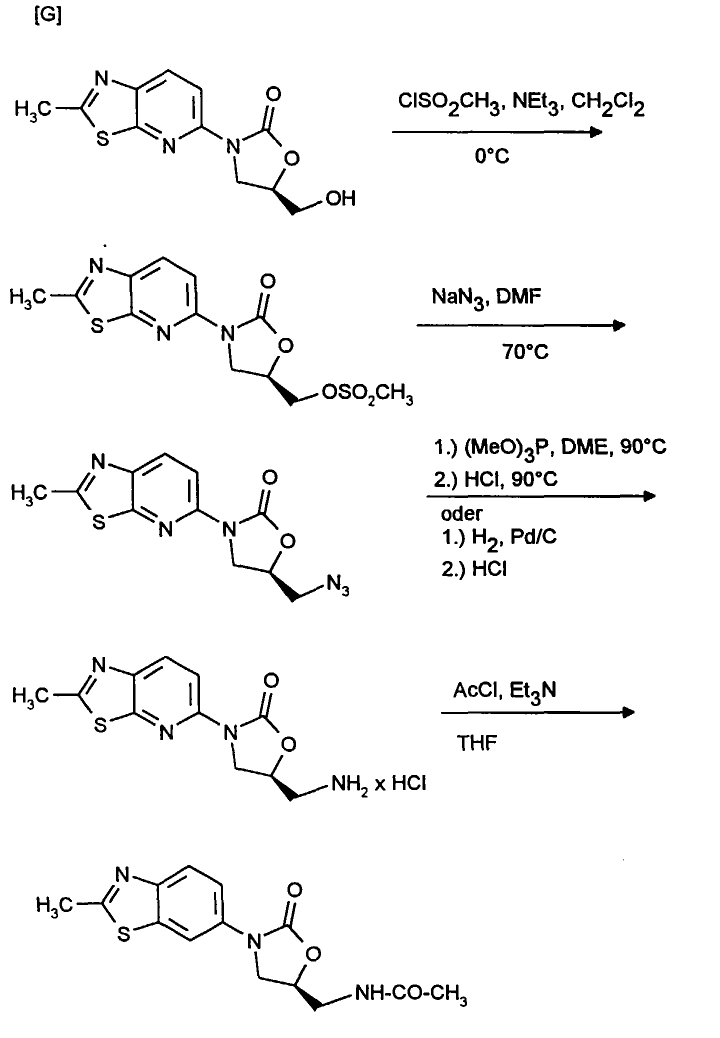

Als Lösemittel eignen sich in Abhängigkeit von den einzelnen Verfahrensschritten die üblichen Lösemittel, die sich Unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formeln (II), (III), (IV) und (Va) eingesetzt.

Alle Umsetzungen werden im allgemeinen bei normalen, erhöhtem oder bei erniedrigtem Druck durchgeführt (z.B. 0,5 bis 5 bar). Im allgemeinen arbeitet man bei Normaldruck.

Das Verfahren [A] erfolgt bevorzugt in Xylol oder Dichlorbenzol, gegebenenfalls in Gegenwart von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [B] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran und Lithium-bis-trimethylsilylamid oder n-Butyllithium, in einem Temperaturbereich von -100°C bis +20°C, vorzugsweise von -75°C bis -40°C.

Für das Verfahren [C] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Als Basen für den 1. Schritt des Verfahren [D] eignen sich im allgemeinen Lithiumalkyle, Lithium-N-alkyle oder Alkalimetallhydride wie beispielsweise Butyllithium oder Natriumhydrid. Bevorzugt ist Natriumhydrid.

Die Base wird im allgemeinen in einer Menge von 1 mol bis 5 mol, bevorzugt von 1 mol bis 1,5 mol bezogen auf 1 mol der Verbindung der allgemeinen Formel (VI) eingesetzt.

Die Cyclisierung erfolgt in einer der oben aufgeführten Lösemitteln und Basen, wobei Kaliumcarbonat und Acetonitril bevorzugt sind.

Die Base wird in einer Menge von 1 mol bis 10 mol, bevorzugt von 1 mol bis 3 mol bezogen auf 1mol der Verbindungen der allgemeinen Formeln (II), (III), (IV) und (Va) eingesetzt.

Alle Umsetzungen werden im allgemeinen bei normalen, erhöhtem oder bei erniedrigtem Druck durchgeführt (z.B. 0,5 bis 5 bar). Im allgemeinen arbeitet man bei Normaldruck.

Die Reaktionsschritte werden im allgemeinen in einem Temperaturbereich von -78°C bis 100°C, bevorzugt von -20°C bis 50°C durchgeführt.

Die Cyclisierung [E] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (VIII) eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester und Chlorameisensäuretrichlormethylester.

Als Lösemittel eignen sich die oben aufgeführten Halogenkohlenwasserstoffe. Bevorzugt ist Methylenchlorid.

Die Cyclisierungen erfolgen im allgemeinen in einem Temperaturbereich von -20°C bis 100°C, vorzugsweise bei -20°C bis Raumtemperatur.

Das Verfahren [F] erfolgt in Analogie zu den unter [E] aufgeführten Bedingungen.

Die Acylierung [G] erfolgt im allgemeinen in einem der oben aufgeführten Ether oder Halogenkohlenwasserstoffe, vorzugsweise Tetrahydrofuran oder Methylenchlorid, in einem Temperaturbereich von -30°C bis 50°C, bevorzugt von -10°C bis Raumtemperatur.

Die Reduktionen erfolgen im allgemeinen mit Hydriden in inerten Lösemitteln oder mit Boranen, Diboranen oder ihren Komplexverbindungen.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Die Reduktion oder Hydrierung der Azide [G] erfolgt mit (CH₃O)₃P und Salzsäure.

Die Reduktion erfolgt im allgemeinen in einem Temperaturbereich von -50°C bis zum jeweiligen Siedepunkt des Lösemittels, bevorzugt von -20°C bis +90°C.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Abspaltung der Hydroxyschutzgruppen erfolgt im allgemeinen nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Abspaltung der Aminoschutzgruppe erfolgt im allgemeinen, ebenfalls nach üblichen Methoden und zwar vorzugsweise Boc mit Salzsäure in Dioxan, Fmoc mit Piperidin und Z mit HBr/HOAc oder durch Hydrogenolyse.

Als bevorzugte Derivatisierungsreaktionen werden Redoxreaktionen, reduktive Aminierung, Umesterung und die Halogenisierung von Methylgruppen mit N-Bromsuccinimid (NBS) oder N-Chlorsuccinimid (NCS) aufgeführt, die im folgenden beispielhaft erläutert werden.

Als Lösemittel für die Alkylierung eignen sich übliche organische Lösemittel, die sich unter den Resktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dichlormethan, Dimethylsulfoxid und Dimethylformamid.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung und die Sulfoamidierung erfolgen im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan oder Erdölfraktionen, Nitromethan, Dimethylformamid, Acetonitril oder Tetrahydrofuran. Ebenso ist es möglich, Gemische der Lösemittel einzusetzen. Besonders bevorzugt sind Dichlormethan und Tetrahydrofuran.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.-buylat, oder organische Amine wie Benzyltrimethylammoniumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder 4-Dimethylaminopyridin.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt wird Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol und Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C und Normaldruck.

Die Verbindungen der allgemeinen Formeln (IV) und (X) sind bekannt oder können nach üblichen Methoden hergestellt werden.

Die Verbindungen der allgemeinen Formel (IX) sind größtenteils neu und können beispielsweise wie oben beschrieben hergestellt werden.

Die Verbindungen der allgemeinen Formel (II) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (III) sind teilweise bekannt oder neu und können dann beispielsweise hergestellt werden, indem man ausgehend von den entsprechenen Carbonsäuren entweder mit Chlorameisensäureisobutylester / Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei -10°C bis Raumtemperatur umsetzt.

Die Verbindungen der allgemeinen Formeln (V) und (Va) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die Verbindungen der allgemeinen Formeln (VI) und (VII) können nach den oben aufgeführten Methoden hergestellt werden.

Die Verbindungen der allgemeinen Formel (Ia) sind neu und können beispielsweise wie unter [A], [B], [C], [D], [E] oder [F] beschrieben hergestellt werden.

Die Verbindungen der allgemeinen Formeln (Ib), (Ic), (Id) und (Ie) sind neu und können wie oben beschrieben hergestellt werden.

Die Verbindungen der allgemeinen Formel (VIII) sind größtenteils bekannt oder neu und können beispielsweise hergestellt werden, indem man ausgehend von den freien Aminen (Ia) entweder mit dem Acetonid von Glycerinaldehyd in Methanol und in Anwesenheit von Natriumacetat / Natriumcyanborhydrid oder von Natriumboranat und Methanol in einem Temperaturbereich von -20°C bis +40°C, bevorzugt von -10°C bis 20°C und Normaldruck umsetzt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

| **MHK-Werte (µg/ml)** | | | | | | | |
|---|---|---|---|---|---|---|---|
| **Bsp.- Nr.** | **Staph. 133** | **Staph. 48N** | **Staph 25701** | **Staph. 9TV** | **E. coli Neumann** | **Klebs. 57 USA** | **Psdm. Bonn** |
| 10 | 8 | 8 | 8 | 4 | >64 | >64 | >64 |
| 11 | 8 | 8 | 8 | 4 | >64 | >64 | >64 |
| 12 | 8 | 8 | 8 | 4 | >64 | >64 | >64 |
| 13 | 8 | 8 | 8 | 4 | >64 | >64 | >64 |
| 14 | 4 | 8 | 4 | 2 | >64 | >64 | >64 |

Für schnellwachsende Mykobakterien wurde die MHK-Bestimmung in Anlehnung an die von Swenson beschriebene Methode der Flüssig-Mikrodilution durchgeführt [vgl. J.M. Swenson, C. Thornberry, U.A. Silcox, Rapidly growing mycobacteria. Testing of susceptibility to 34 antimicrobial agents by broth microdilution. Antimicrobial Agents and Chemotherapy Vol, 22, 186-192 (1982)]. Abweichend davon war das mit 0,1 Vol.% Tween 80 versetzte Hirn-Herzextrakt Medium.

Die verwendeten Mykobakterienstämme wurden von der DSM (Dt. Sammlung von Mikroorganismen, Braunschweig) bezogen. Sie wurden in einer feuchten Kammer bei 37°C bebrütet.
Die MHK-Werte wurden nach 2-4 Tagen abgelesen, wenn die präparatfreien Kontrollen durch Wachstum trüb waren. Der MHK-Wert definiert sich als die niedrigste Präparatkonzentration, die makroskopisch sichtbares Wachstum völlig inhibiert.

| **MHK: Mycobacterium smegmatis** | | |
|---|---|---|
| **Keim:** | **DSM 43061** | **DSM 43465** |
| Bsp.-Nr. | | |
| 10 | 4 | 4 |
| 11 | 8 | 4 |
| 12 | 4 | 4 |
| 13 | 8 | 8 |
| Isoniazid | 4 | 1 |
| Streptomycin | 4 | 4 |

Die erfindungsgemäßen Verbindungen der allgemeinen Formeln (I), (Ia), (Ib), (Ic), (Id) und (Ie) weisen bei geringer Toxizität ein breites antibakterielles Spektrum, speziell gegen gram-positive Bakterien, Haemophilus influenzae, anaerobe Keime und schnellwachsende Mykobakterien auf. Diese Eigenschaften ermöglichen ihre Verwendung als chemotherapeutische Wirkstoffe in der Human- und Tiermedizin.

Besonders wirksam sind die erfindungsgemäßen Verbindungen gegen Bakterien und bakterienähnliche Mikroorganismen wie Mycoplasmen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die erfindungsgemäßen Verbindungen können zum Zweck der Erweiterung des Wirkungsspektrums und um eine Wirkungssteigerung zu erreichen auch mit anderen Antibiotika kombiniert werden.

### Verwendete Laufmittelgemische

### I Dichlormethan : Methanol

### Ausgangsverbindungen

### Beispiel I

### 2-Methyl-6-nitrooxazolo[4,5-b]pyridin-2(3H)-on

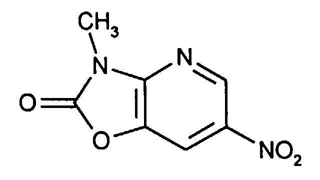

25 g (138 mmol) 6-Nitrooxazolo[4,5-b]pyridin-2(3H)-on (Helv. Chim. Acta 1976, 59, 1593) und 31 ml (207 mmol) Diazabicycloundecen (DBU) in 800 ml DMF werden 1 h bei 50°C gerührt. Anschließend werden 86,7 ml (1,38 mmol) Iodmethan zugetropft und das Reaktionsgemisch 16 h bei 100°C gerührt. Zur Aufarbeitung wird das DMF im Vakuum abgezogen, der Rückstand mit Dichlormethan versetzt, das unlösliche Produkt abgesaugt und getrocknet.
Ausbeute: 21,4 g (79% d.Th.)
¹H-NMR (200 MHz, [D₆]DMSO): δ = 9,0 (d, 1H); 8,54 (d, 1H); 3,40 (s, 3H).

### Beispiel II

### 6-Amino-3-methyloxazolo[4,5-b]pyridin-2(3H)-on

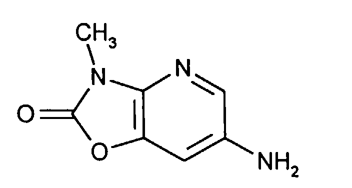

1,56 g (8 mmol) der Verbindung aus Beispiel I und 450 mg Pd-C (10%) in 100 ml Methanol werden 6 h unter Wasserstoff (1 atm) gerührt. Es wird vom Katalysator abfiltriert, das Lösemittel abgezogen und getrocknet.
Ausbeute: 1,2 g (91% d.Th.)
¹H-NMR (200 MHz, [D₆]DMSO): δ = 7,50 (d, 1H); 6,98 (d, 1H); 5,20 (bs 1H); 3,30 (s, 3H).

### Beispiel III

### 6-Benzyloxycarbonylamino-3-methyloxazolo[4,5-b]pyridin-2(3H)-on

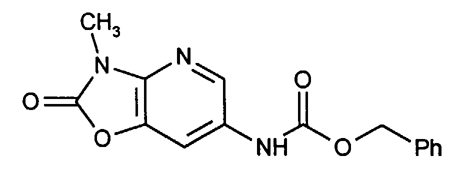

7,1 g (42,9 mmol) der Verbindung aus Beispiel II in 300 ml THF und 40 ml ges. NaHCO₃-Lösung werden bei 0°C tropfenweise mit 6,7 ml (47,19 mmol) Chlorameisensäurebenzylester versetzt. Nach 1 h wird 1 l Wasser hinzugegeben, der Niederschlag abgesaugt, mit Wasser und Petrolether gewaschen und getrocknet.
Ausbeute: 12,4 g (96% d.Th.)
R_{f} (I, 10:1) = 0,66
¹H-NMR (200 MHz, [D₆]DMSO): δ = 9,95 (bs, 1H); 8,10 (d, 1H); 7,80 (d, 1H); 7,30 - 7,50 (m, 5H); 5,18 (s, 2H); 3,28 (s, 3H).

### Beispiel IV

### 6-(N-Allyl-N-benzyloxycarbonylamino)-3-methyloxazolo[4,5-b]pyridin-2(3H)-on

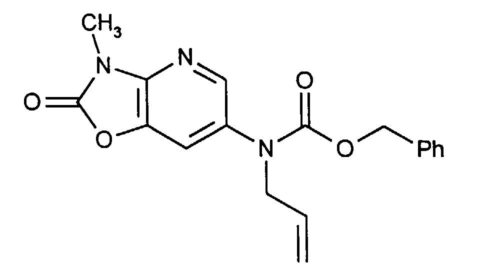

Zu einer Lösung von 8 g (26,7 mmol) der Verbindung aus Beispiel III in 300 ml DMF werden 0,7 g (29 mmol) Natriumhydrid (80% in Paraffin) gegeben und das Reaktionsgemisch 1 h bei Raumtemperatur gerührt. Anschließend versetzt man mit 2,5 ml (29 mmol) Allylbromid und rührt weitere 2 h bei Raumtemperatur. Man gibt auf 800 ml Wasser, extrahiert die wäßrige Phase mit Diethylether, trocknet (Na₂SO₄) und zieht im Vakuum die Lösemittel ab. Das Rohprodukt wird aus tert.Butylethylether umkristallisiert.
Ausbeute: 7,42 g (82% d.Th.)
R_{f} (I, 10:1) = 0,30
¹H-NMR (200 MHz, [D₆]DMSO): δ = 8,10 (d, 1H); 7,80 (d, 1H); 7,20 - 7,45 (m, 5H); 5,79 - 5,95 (m, 1H); 5,00 - 5,20 (m, 4H); 4,28 (d, 2H); 3,30 (s, 3H).

### Beispiel V

### 6-(N-Benzyloxycarbonyl-N-(2,3-dihydroxyprop-1-yl)amino)-3-methyloxazolo[4,5-b]pyridin-2(3H)-on

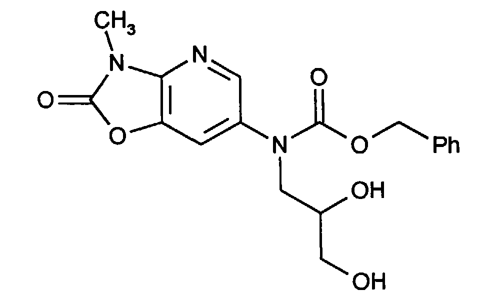

Eine Lösung von 7,27 g (21,4 mmol) der Verbindung aus Beispiel IV und 14,9 g (128,2 mmol) N-Methylmorpholin-N-oxid in 400 ml Aceton und 100 ml Wasser wird mit 23,6 ml einer Lösung von Osmiumtetroxid (2,5% in Wasser) versetzt, und das Reaktionsgemisch 16 h bei Raumtemperatur gerührt. Die Lösung wird auf 0°C gekühlt, mit 240 ml NaHSO₃-Lösung (39%ig) versetzt und weitere 2 h bei Raumtemperatur gerührt. Das Reaktionsgemisch wird mit Wasser und ges. NaCl-Lösung (1:1) versetzt, die wäßrige Phase mit Essigester extrahiert und die vereinigten organischen Phasen getrocknet (Na₂SO₄). Nach Abziehen der Lösemittel im Vakuum erhält man die Titelverbindung als gelben Feststoff.
Ausbeute: 8,1 g (quant.)
R_{f} (I, 10:1) = 0,32
¹H-NMR (200 MHz, [D₆]DMSO): δ = 8,14 (d, 1H); 7,80 (d, 1H) ; 7,10 - 7,45 (m, 5H); 5,10 (bs, 2H); 4,90 (d, 1H); 4,54 (t, 1H); 3,40 - 3,80 (m, 3H); 3,28 (s, 3H).

### Beispiel VI

### 6-Acetylamino-2-methylthiazolo[5,4-b]pyridin

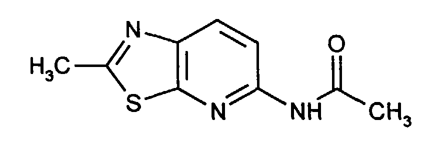

2,85 g (20,2 mmol) 2,6-Diaminothiazolo[5,4-b]pyridin (vgl. J. Org. Chem. **1973**, 38, 4383) in 4,3 ml Essigsäureanhydrid werden 2 h unter Rückfluß erhitzt. Das Reaktionsgemisch wird eingeengt, und der Rückstand mit 1 M NaOH-Lösung alkalisch gestellt. Man extrahiert mit Essigester, trocknet (MgSO₄) und zieht im Vakuum die Lösemittel ab.
Ausbeute: 2,71 g (65% d.Th.)
R_{f} (I, 10:1) = 0,53

### Beispiel VII

### 6-Amino-2-methylthiazolo[5,4-b]pyridin

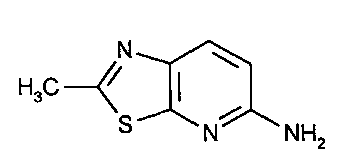

1,33 g (5,96 mmol) der Verbindung aus Beispiel VI in 17,3 ml konzentrierter Salzsäure werden 1 h unter Rückfluß erhitzt. Zur Aufarbeitung wird das Reaktionsgemisch im Vakuum eingeengt und mit 1 M NaOH-Lösung auf pH = 9 gebracht und mit Essigester extrahiert. Die vereinigten organischen Phasen werden getrocknet (MgSO₄) und im Vakuum das Lösemittel abgezogen.
Ausbeute: 0,8 g (81% d.Th.)
R_{f} (I, 10:1) = 0,58

### Beispiel VIII

### 6-Isocyanato-2-methylthiazolo[5,4-b]pyridin-hydrochlorid

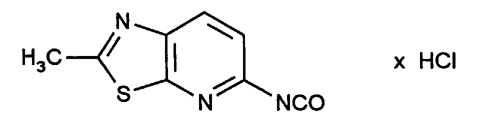

1,01 g (6,1 mmol) der Verbindung aus Beispiel VII und 810 µl (6,71 mmol) Chlorameisensäuretrichlormethylester in 10 ml Dichlorethan werden 16 h unter Rückfluß erhitzt. Man läßt auf Raumtemperatur abkühlen, saugt den entstandenen Niederschlag ab, wäscht mit Dichlormethan und trocknet im Hochvakuum.
Ausbeute: 1.07 g (77% d.Th.)

### Herstellungsbeispiele

### Beispiel 1

### 5-Hydroxymethyl-3-(3-methyloxazolo[4,5-b]pyridin-2(3H)-on-6-yl)oxazolidin-2-on

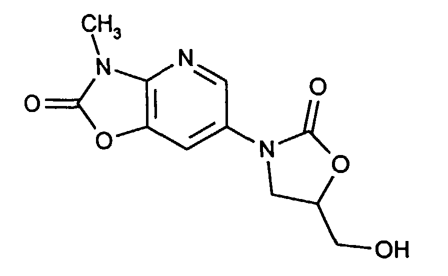

7,75 g (20.8 mmol) der Verbindung aus Beispiel V und 5,8 g (42 mmol) Kaliumcarbonat in 500 ml Acetonitril werden 15 h unter Rückfluß erhitzt. Zur Aufarbeitung wird das Reaktionsgemisch in Wasser gegossen, die wäßrige Phase mit Natriumchlorid gesättigt und mit Essigester extrahiert. Die vereinigten organischen Phasen werden getrocknet (Na₂SO₄), die Lösemittel im Vakuum abgezogen und der Rückstand mit Diethylether kristallisiert.
Ausbeute: 3,14 g (57% d.Th.)
R_{f} (I, 10:1) = 0.83
MS (CI): m/z = 283 (M+NH₄⁺)
¹H-NMR (200 MHz, [D₆]DMSO): δ = 8,20 (d, 1H); 8.02 (d, 1H); 5,22 (t, 1H, OH); 4,75 (m, 1H); 4,10 (t, 1H); 3,85 (dd, 1H); 3,49 - 3,76 (m, 2H); 3,30 (s, 3H).

### Beispiel 2

### (5R)-5-Butyoxymethyl-3-(2-methylthiazolo[5,4-b]pyridin-2-yl)oxazolidin-2-on

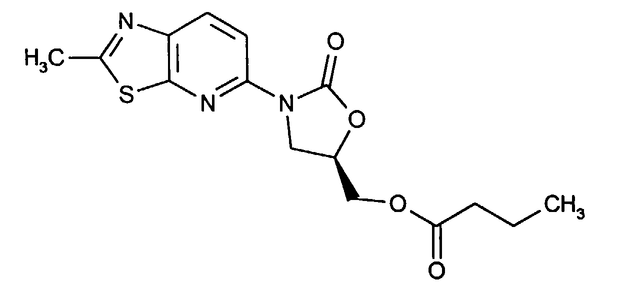

Eine Suspension von 29,0 mg (0,33 mmol) Lithiumbromid und 72,0 mg (0,33 mmol) Tributylphosphinoxid in 10 ml Xylol wird 1 h am Wasserabscheider unter Rückfluß erhitzt. Anschließend gibt man in der Siedehitze 776 µl (5,6 mmol) Triethylamin und 782 µl (5,6 mmol) Glycidylbutyrat, gefolgt von 1,27 g (5,6 mmol) der Verbindung aus Beispiel VII hinzu und rührt weitere 3 h unter Rückfluß. Man läßt auf Raumtemperatur abkühlen, zieht im Vakuum die Lösemittel ab und reinigt den Rückstand durch Chromatographie (Kieselgel, Laufmittel Dichlormethan / Methanol 30:1)
Ausbeute: 464 mg (25% d.Th.)
R_{f} (I, 10:1) = 0,9
MS (CI): m/z = 336 (M+H)⁺
¹H-NMR (200 MHz, [D₆]DMSO): 8,33 (d, 1H); 8,22 (d, 1H); 4,98 (m, 1H); 4,35 (m, 3H); 4,12 (dd, 1H); 2,80 (s, 3H); 2,28 (t, 2H); 1,50 (sextett, 2H); 0,80 (t, 3H).

### Beispiel 3

### (5R)-5-Hydroxymethyl-3-(2-methylthiazolo[5,4-b]pyridin-6-yl)oxazolidin-2-on

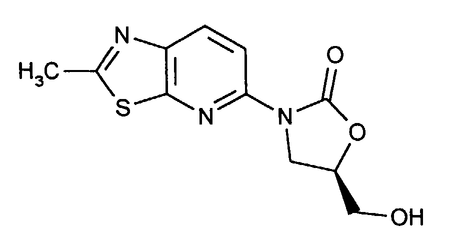

424 mg (1,26 mmol) der Verbindung aus Beispiel 2 in 30 ml Methanol werden mit 35 mg (0,1 mmol) Caesiumcarbonat versetzt und 3 h bei Raumtemperatur gerührt. Das Reaktionsgemisch wird mit Essigester versetzt, mit ges. NH₄Cl-Lösung und Wasser gewaschen, getrocknet (MgSO₄) und im Vakuum die Lösemittel abgezogen.
Ausbeute: 245 mg (73% d.Th.)
R_{f} (I, 30:1) = 0.43
MS(CI): m/z = 266 (M+H)⁺
¹H-NMR (200 MHz, [D₆]DMSO): δ = 8,33 (d, 1H); 8,25 (d, 1H); 5,23 (t, 1H); 4,75 (m, 1H); 4,25 (t, 1H); 4,05 (dd, 1H); 3,65 (m, 2H); 2,80 (s, 3H).

### Beispiel 4

### 5-Methansulfonyloxy-3-(3-methyloxazolo[4,5-b]pyridin-2(3H)-on-6-yl)oxazolidin-2-on

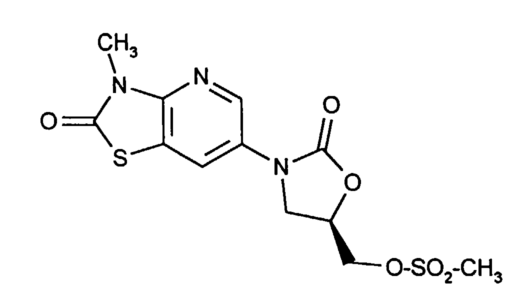

Eine Mischung aus 3,0 g (113 mmol) der Verbindung aus Beispiel 1, 2,7 ml (19,2 mmol) Triethylamin in 120 ml THF wird bei 0°C mit 1,4 ml (18,1 mmol) Methansulfonsäurechlorid versetzt und 1 h bei 0°C gerührt. Das Reaktionsgemisch wird auf 1,2 l Eiswasser gegeben, der entstandene Niederschlag abgesaugt, mit Wasser und Petrolether gewaschen und im Hochvakuum über P₂O₅ getrocknet.
Ausbeute: 3,1 g (80% d.Th.)
R_{f} (I, 10:1) = 0,58
¹H-NMR (200 MHz, [D₆]DMSO): δ = 8,22 (d, 1H); 8,02 (d, 1H); 5,05 (m, 1H); 4,45 - 4,60 (m, 2H); 4,23 (t, 1H); 3,70 (dd, 1H); 3,30 (s, 3H); 3,32 (s, 3H).

In Analogie zur Vorschrift des Beispiels 4 wird die in der Tabelle 1 aufgeführten Verbindung hergestellt:

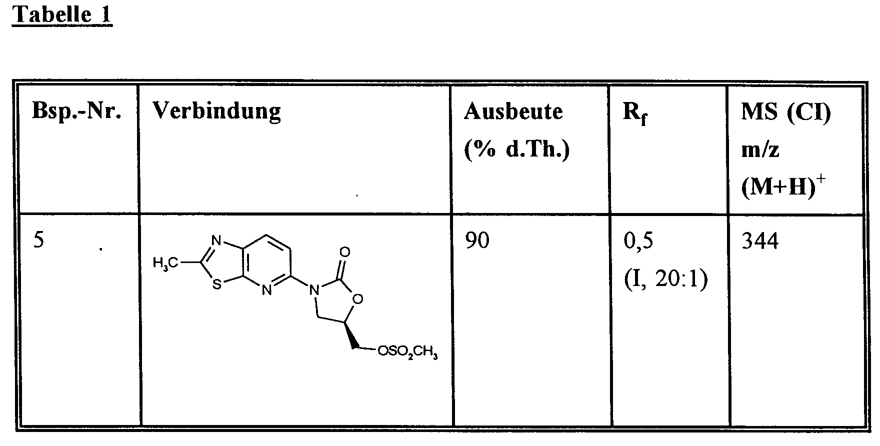

### Beispiel 6

### 5-Azidomethyl-3-(3-methyloxazolo[4,5-b]pyridin-2(3H)-on-6-yl)oxazolidin-2-on

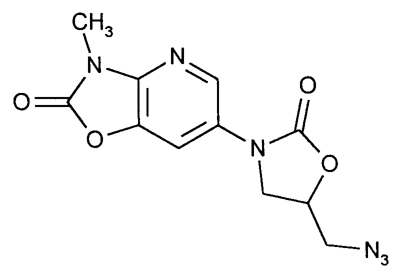

Eine Lösung von 2,95 g (8,6 mmol) der Verbindung aus Beispiel 4 und 0,62 g (9,5 mmol) Natriumazid in 150 ml DMF wird 3 h bei 90°C gerührt. Man läßt auf Raumtemperatur abkühlen, zieht im Vakuum das DMF ab, versetzt den Rückstand mit Wasser und Essigester und extrahiert die organische Phase mit Essigester. Die vereinigten organischen Phasen werden getrocknet (Na₂SO₄) und das Lösemittel im Vakuum abgezogen.
Ausbeute: 2,2 g (88% d.Th.)
R_{f} (I, 20:1) = 0,55
¹H-NMR (200 MHz, [D₆]DMSO): δ = 8,25 (d, 1H); 8,05 (d, 1H); 4,92 (m, 1H); 4,15 (t, 1H); 3,32 (dd, 1H); 3,15 - 3,30 (m, 2H); 3,30 (s, 3H).

In Analogie zur Vorschrift des Beispiels 6 wird die in der Tabelle 2 aufgeführten Verbindung hergestellt:

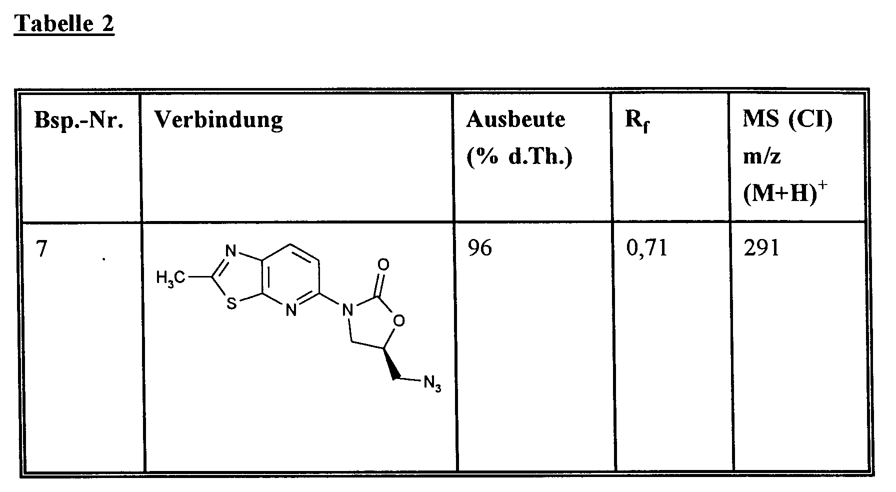

### Beispiel 8

### 5-Aminomethyl-3-(3-methyloxazolo[4,5-b]pyridin-2(3H)-on-6-yl)oxazolidin-2-on

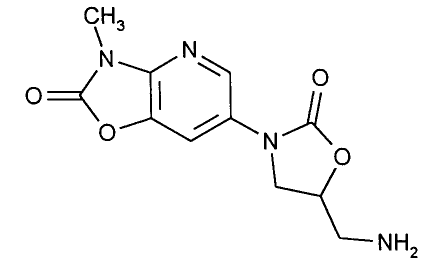

2,15 g (7,4 mmol) der Verbindung aus Beispiel 6 und 200 ml Pd-C (10%) werden in 50ml THF und 50 ml Methanol 1 h unter Wasserstoff (1 atm) gerührt. Nach beendeter Reaktion wird vom Katalysator abfiltriert, die Lösemittel abgezogen und der Rückstand im Hochvakuum getrocknet.
Ausbeute. 1,96 g (quant.)
R_{f} (I, 5:1) = 0,17
MS (CI): m/z = 282 (M+NH₄⁺)
¹H-NMR (200 MHz, [D₆]DMSO): δ = 8,21 (d, 1H); 8,06 (d, 1H); 4,63 (m, 1H); 4,09(t, 1H); 3,90 (dd, 1H); 3,32 (s, 3H); 3,30 - 3,45 (bs, 2H).

### Beispiel 9

### (5S)-5-Aminomethyl-3-(2-methylthiazolo[5,4-b]pyridin-2-yl)oxazolidin-2-on-hydrochlorid

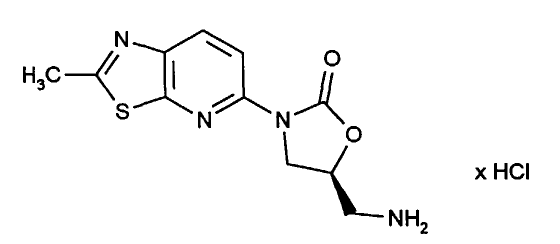

Eine Lösung von 210 mg (0,72 mmol) der Verbindung aus Beispiel 7 in 5 ml Dimethoxyethan (DME) wird bei 50°C tropfenweise mit 0,1 ml (0,84 mmol) Trimethylphosphit versetzt und weitere 3 h bei 50°C gerührt. Man gibt erneut 0,1 ml (0,84 mmol) Trimethylphosphit hinzu, rührt weitere 0,5 h, versetzt das Reaktionsgemisch mit 0,4 ml (2,5 mmol) 6 n HCl und rührt weitere 2 h bei 60°C. Man läßt auf Raumtemperatur abkühlen, versetzt mit Essigester und ges. NaHCO₃-Lösung, extrahiert die wäßrige Phase mit Essigester, trocknet die vereinigten organischen Phasen (MgSO₄) und zieht im Vakuum die Lösemittel ab. Der Rückstand wird in Diethylether aufgenommen und mit einem Überschuß 1M etherische HCl versetzt. Der Niederschlag wird abgesaugt, mit Diethylether gewaschen und getrocknet.
Ausbeute: 105 mg (49% d.Th.)
¹H-NMR (200 MHz, [D₆]DMSO): δ = 8,32 (d, 1H); 8,23 (d, 1H); 4,97 (m, 1H); 4,36 (t, 1H); 4,03 (dd, 1H); 3,25 (m, 2H); 2,80 (s, 3H).

### Beispiel 10

### 5-Acetylaminomethyl-3-(3-methyloxazolo[4,5-b]pyridin-2(3H)-on-6-yl)oxazolidin-2-on

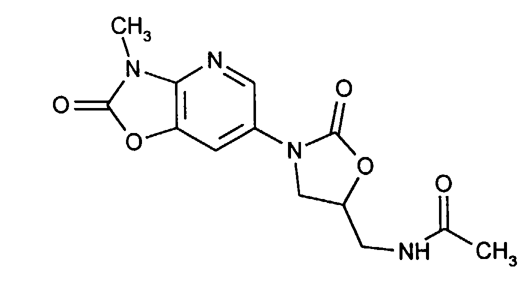

Zu einer Lösung von 150 mg (0,57 mmol) der Verbindung aus Beispiel 9 und 126 ml (0,91 mmol) Triethylamin in 10 ml Dichlormethan werden bei 0°C 61 µl (0,86 mmol) Acetylchlorid gegeben und weitere 1 h bei 0°C gerührt. Das Reaktionsgemisch wird auf 110 ml Eiswasser gegeben, die wäßrige Phase mit Dichlormethan extrahiert, die vereinigten organischen Phasen mit ges. NaCl-Lösung gewaschen, getrocknet (Na₂SO₄), und das Lösemittel im Vakuum abgezogen. Der Rückstand wird mit wenig Dichlormethan kristallisiert.
Ausbeute: 46 mg (27% d.Th.)
R_{f} (I, 20:1) = 0,16
MS (CI): m/z = 324 (M+NH₄⁺)
¹H-NMR (200 MHz, [D₆]DMSO): δ = 8,21 (bt, 1H, NH); 8,15 (d, 1H); 8,02 (d, 1H); 4,74 (m, 1H); 4,15 (t, 1H); 3,73 (dd, 1H); 3,33 (s, 3H); 1,82 (s, 3H).

In Analogie zur Vorschrift des Beispiels 10 werden die in Tabelle 3 aufgeführten Verbindungen hergestellt:

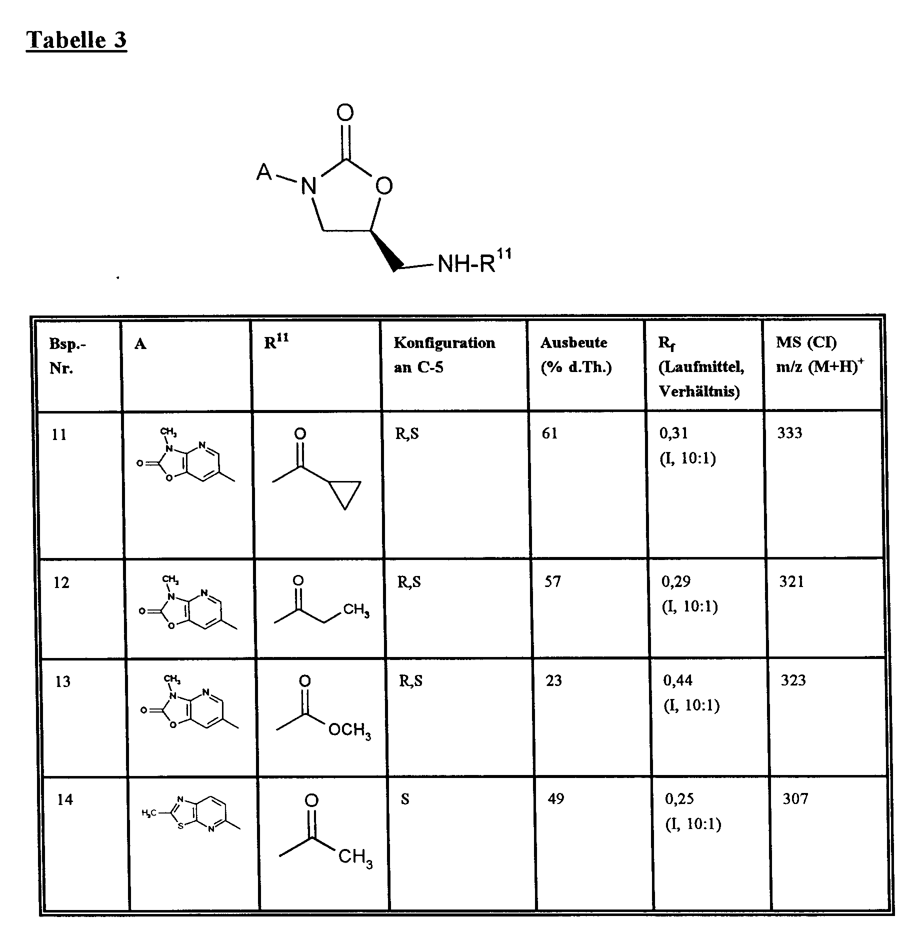

## Patentansprüche

1. Verbindungen der allgemeinen Formel (I) 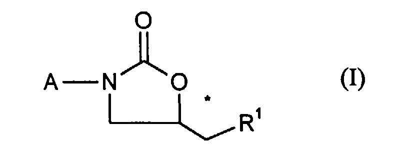 in welcher
A für einen Rest der Formel 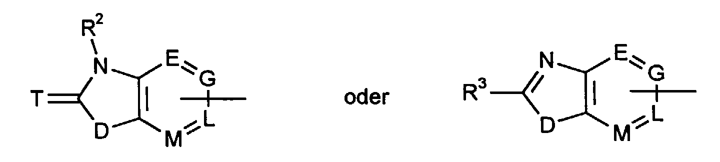 steht,
worin
E, G, L und M gleich oder verschieden sind und mindestens einer der Substituenten ein Stickstoffatom bedeutet und die anderen den Rest der Formel -CR⁴ bedeuten,
worin
R⁴ Wasserstoff, Methyl oder Halogen bedeutet,
R² Wasserstoff, Cycloalkyl oder Cycloalkylcarbonyl mit jeweils 3 bis 8 Kohlenstoffatomen bedeutet, oder geradkettiges oder verzweigtes Alkyl mit bis zu 8 Kohlenstoffatomen bedeutet, das gegebenenfalls durch Hydroxy, Halogen, durch geradkettiges oder verzweigtes Alkoxy, Alkoxycarbonyl oder Alkylthio mit jeweils bis zu 6 Kohlenstoffatomen oder durch einen Rest der Formel -NR⁶R⁷ substituiert ist,
worin
R⁶ und R⁷ gleich oder verschieden sind und Wasserstoff, Cycloalkyl, Phenyl oder geradkettiges oder verzweigtes Alkyl mit bis zu 4 Kohlenstoffatomen bedeuten,
R³ geradkettiges oder verzweigtes Alkyl oder Thioalkyl mit jeweils bis zu 8 Kohlenstoffatomen bedeutet,
D ein Sauerstoff- oder Schwefelatom oder eine Gruppe der Formel -NR⁵ bedeutet,
worin
R⁵ die oben angegebene Bedeutung von R² hat und mit dieser gleich oder verschieden sind,
T bedeutet ein Sauerstoff- oder Schwefelatom,
R¹ für Azido, Hydroxy oder für eine Gruppe der Formel -OR⁸, O-SO₂R⁹ oder -NR¹⁰R¹¹ steht,
worin
R⁸ geradkettiges oder verzweigtes Acyl mit bis zu 8 Kohlenstoffatomen oder eine Hydroxyschutzgruppe bedeutet,
R⁹ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
R¹⁰ und R¹¹ gleich oder verschieden sind und
Cycloalkyl mit 3 bis 6 Kohlenstoffatomen, Wasserstoff, Phenyl oder geradkettiges oder verzweigtes Alkyl oder Alkoxy mit jeweils bis zu 8 Kohlenstoffatomen oder eine Aminoschutzgruppe bedeuten,
oder
R¹⁰ oder R¹¹ eine Gruppe der Formel -CO-R¹², -CS-R^{12'}, P(O)(OR¹³)(OR¹⁴) oder -SO₂-R¹⁵ bedeutet,
worin
R¹² und R^{12'} gleich oder verschieden sind und
Wasserstoff, Cycloalkyl mit 3 bis 6 Kohlenstoffatomen, Trifluormethyl, geradkettiges oder verzweigtes Alkoxy mit bis zu 8 Kohlenstoffatomen, Phenyl, Benzyloxy oder Wasserstoff bedeuten, oder
geradkettiges oder verzweigtes Alkyl mit bis zu 8 Kohlenstoffatomen bedeuten, das gegebenenfalls durch Cyano, Halogen oder Trifluormethyl substituiert ist,
oder
geradkettiges oder verzweigtes Thioalkyl oder Acyl mit jeweils bis zu 6 Kohlenstoffatomen bedeuten,
oder
eine Gruppe der Formel -NR¹⁶R¹⁷ bedeuten,
worin
R¹⁶ und R¹⁷ gleich oder verschieden sind und Wasserstoff, Phenyl oder geradkettiges oder verzweigtes Alkyl mit bis zu 6 Kohlenstoffatomen bedeuten,
oder
einen 5-gliedrigen aromatischen Heterocyclus mit bis zu 3 Heteroatomen aus der Reihe S, N und/oder O bedeuten,
R¹³ und R¹⁴ gleich oder verschieden sind und Wasserstoff oder geradkettiges oder verzweigtes Alkyl mit bis zu 4 Kohlenstoffatomen bedeuten,
R¹⁵ geradkettiges oder verzweigtes Alkyl mit bis zu 4 Kohlenstoffatomen oder Phenyl bedeutet,
als reine Stereoisomere oder als Stereoisomeren-Gemische, und deren Salze.

2. Verbindungen der allgemeinen Formel (I) gemäß Anspruch 1, in welcher
A für einen Rest der Formel 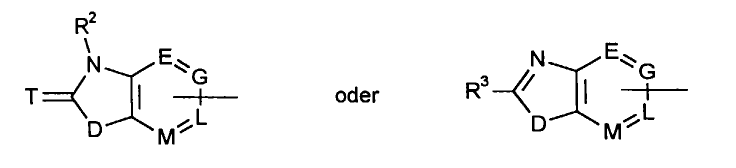 steht,
worin
E, G, L und M gleich oder verschieden sind und mindestens einer der Substituenten ein Stickstoffatom bedeutet und die anderen den Rest der Formel -CR⁴ bedeuten,
worin
R⁴ Wasserstoff, Fluor, Chlor oder Brom bedeutet,
R² Wasserstoff, Cyclopropyl, Cyclobutyl, Cyclopentyl, Cyclohexyl oder Cycloheptyl bedeutet, oder
geradkettiges oder verzweigtes Alkyl mit bis zu 6 Kohlenstoffatomen bedeutet, das gegebenenfalls durch Hydroxy, Fluor, Chlor, Brom, durch geradkettiges oder verzweigtes Alkoxy, Alkoxycarbonyl oder Alkylthio mit jeweils bis zu 4 Kohlenstoffatomen oder durch einen Rest der Formel -NR⁶R⁷ substituiert ist,
worin
R⁶ und R⁷ gleich oder verschieden sind und
Wasserstoff, Cyclopropyl, Cyclobutyl, Cyclopentyl, Cylcohexyl, Phenyl oder geradkettiges oder verzweigtes Alkyl mit bis zu 3 Kohlenstoffatomen bedeuten,
R³ geradkettiges oder verzweigtes Alkyl oder Thioalkyl mit jeweils bis zu 6 Kohlenstoffatomen bedeutet,
D ein Sauerstoff- oder Schwefelatom oder eine Gruppe der Formel -NR⁵ bedeutet,
worin
R⁵ die oben angegebene Bedeutung von R² hat und mit dieser gleich oder verschieden ist,
T ein Sauerstoff- oder Schwefelatom bedeutet,
R¹ für Azido, Hydroxy oder für eine Gruppe der Formel -OR⁸, O-SO₂R⁹ oder -NR¹⁰R¹¹ steht,
worin
R⁸ geradkettiges oder verzweigtes Acyl mit bis zu 6 Kohlenstoffatomen oder Benzyl bedeutet,
R⁹ geradkettiges oder verzweigtes Alkyl mit bis zu 3 Kohlenstoffatomen, Phenyl oder Toluolyl bedeutet
R¹⁰ und R¹¹ gleich oder verschieden sind und
Cyclopropyl, Cyclopentyl, Cyclohexyl, Wasserstoff, Phenyl oder geradkettiges oder verzweigtes Alkyl oder Alkoxy mit jeweils bis zu 6 Kohlenstoffatomen, tert.Butoxycarbonyl oder Benzyloxycarbonyl bedeuten,
oder
R¹⁰ oder R¹¹ eine Gruppe der Formel -CO-R¹², -CS-R^{12'}, P(O)(OR¹³)(OR¹⁴) oder -SO₂-R¹⁵ bedeutet,
worin
R¹² und R^{12'} gleich oder verschieden sind und
Wasserstoff, Cyclopropyl, Cyclobutyl, Cyclopentyl, Cyclohexyl, Trifluormethyl oder geradkettiges oder verzweigtes Alkoxy mit bis zu 6 Kohlenstoffatomen, Phenyl, Benzyloxy oder Wasserstoff bedeuten, oder
geradkettiges oder verzweigtes Alkyl mit bis zu 6 Kohlenstoffatomen bedeuten, das gegebenenfalls durch Cyano, Fluor, Chlor, Brom oder Trifluormethyl substituiert ist, oder
geradkettiges oder verzweigtes Thioalkyl oder Acyl mit jeweils bis zu 5 Kohlenstoffatomen bedeuten, oder
eine Gruppe der Formel -NR¹⁶R¹⁷ bedeuten,
worin
R¹⁶ und R¹⁷ gleich oder verschieden sind und Wasserstoff, Phenyl oder geradkettiges oder verzweigtes Alkyl mit bis zu 4 Kohlenstoffatomen bedeuten, oder
Isoxazolyl, Furyl, Thienyl, Pyrryl, Oxazolyl oder Imidazolyl bedeuten,
R¹³ und R¹⁴ gleich oder verschieden sind und Wasserstoff oder geradkettiges oder verzweigtes Alkyl mit bis zu 3 Kohlenstoffatomen bedeuten,
R¹⁵ geradkettiges oder verzweigtes Alkyl mit bis zu 3 Kohlenstoffatomen oder Phenyl bedeutet,
als reine Stereoisomere oder als Stereoisomerengemische, und deren Salze.

3. Verbindungen der allgemeinen Formel (I) gemäß Anspruch 1,
in welcher
A für einen Rest der Formel 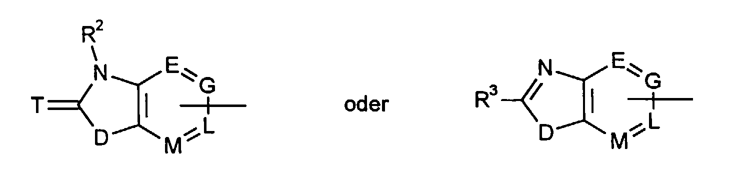 steht,
worin
E, G, L und M gleich oder verschieden sind und mindestens einer der Substituenten ein Stickstoffatom bedeutet und die anderen den Rest der Formel -CR⁴ bedeuten,
worin
R⁴ Wasserstoff oder Fluor bedeutet,
R² Wasserstoff, Cyclopropyl, Cyclobutyl, Cyclopentyl oder Cyclohexyl bedeutet, oder
geradkettiges oder verzweigtes Alkyl mit bis zu 5 Kohlenstoffatomen bedeutet, das gegebenenfalls durch Hydroxy, Fluor, Chlor, Brom, durch geradkettiges oder verzweigtes Alkoxy, Alkoxycarbonyl oder Alkylthio mit jeweils bis zu 3 Kohlenstoffatomen oder durch einen Rest der Formel -NR⁶R⁷ substituiert ist,
worin
R⁶ und R⁷ gleich oder verschieden sind und
Wasserstoff, Cyclopropyl, Cyclopentyl, Cyclohexyl, Phenyl oder geradkettiges oder verzweigtes Alkyl mit bis zu 3 Kohlenstoffatomen bedeuten,
R³ geradkettiges oder verzweigtes Alkyl oder Thioalkyl mit jeweils bis zu 5 Kohlenstoffatomen bedeutet,
D ein Sauerstoff- oder Schwefelatom bedeutet,
T ein Sauerstoffatom bedeutet,
R¹ für Azido, Hydroxy oder für eine Gruppe der Formel -OR⁸, O-SO₂R⁹ oder -NR¹⁰R¹¹ steht,
worin
R⁸ geradkettiges oder verzweigtes Acyl mit bis zu 5 Kohlenstoffatomen oder Benzyl bedeutet,
R⁹ Methyl, Ethyl, Phenyl oder Tolyl bedeutet,
R¹⁰ und R¹¹ gleich oder verschieden sind und
Cyclopropyl, Cyclopentyl, Cyclohexyl, Wasserstoff, Phenyl oder geradkettiges oder verzweigtes Alkyl oder Alkoxy mit jeweils bis zu 5 Kohlenstoffatomen, tert.Butoxycarbonyl oder Benzyloxycarbonyl bedeuten,
oder
R¹⁰ oder R¹¹ eine Gruppe der Formel -CO-R¹², -CS-R^{12'}, P(O)(OR¹³)(OR¹⁴) oder -SO₂R¹⁵ bedeutet,
worin
R¹² und R^{12'} gleich oder verschieden sind und
Wasserstoff, Cyclopropyl, Cyclobutyl, Cyclopentyl, Trifluormethyl oder geradkettiges oder verzweigtes Alkoxy mit bis zu 5 Kohlenstoffatomen, Phenyl, Benzyloxy oder Wasserstoff bedeuten,
geradkettiges oder verzweigtes Alkyl mit bis zu 5 Kohlenstoffatomen bedeuten, das gegebenenfalls durch Cyano, Fluor, Chlor, Brom oder Trifluormethyl substituiert ist, oder
geradkettiges oder verzweigtes Thioalkyl- oder Acyl mit jeweils bis zu 4 Kohlenstoffatomen bedeuten, oder
eine Gruppe der Formel -NR¹⁶R¹⁷ bedeuten,
worin
R¹⁶ und R¹⁷ gleich oder verschieden sind und Wasserstoff, Phenyl oder geradkettiges oder verzweigtes Alkyl mit bis zu 3 Kohlenstoffatomen bedeuten, oder
Isoxazolyl, Furyl, Oxazolyl oder Imidazolyl bedeuten,
R¹³ und R¹⁴ gleich oder verschieden sind und Wasserstoff, Methyl oder Ethyl bedeuten,
R¹⁵ Methyl oder Phenyl bedeutet
als reine Stereoisomere oder als Stereoisomeren-Gemische, und deren Salze.

4. Verbindungen der allgemeinen Formel (I) gemäß Anspruch 1,
in welcher
der Oxazolidinonrest in den Positionen 5 oder 6 an den stickstoffhaltigen Ring angebunden ist.

5. Verfahren zur Herstellung von Verbindungen der allgemeinen Formel (I) gemäß Anspruch 1, dadurch gekennzeichnet, daß man
[A] Verbindungen der allgemeinen Formel (II) oder (III)
A-N=C=O (II)
oder A-CO-N₃ (III)
in welchen
A die in Anspruch 1 angegebene Bedeutungen hat,
mit Lithiumbromid/(C₄H₉)₃P(O) und Epoxiden der allgemeinen Formel (IV) 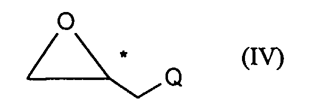 in welcher
Q für C₁-C₆-Acyloxy steht,
in inerten Lösemitteln, gegebenenfalls in Anwesenheit einer Base
umsetzt,
und im Fall R¹ = OH durch eine typische Esterverseifung oder durch eine typische Umesterung die Hydroxyfunktion freisetzt,
oder
[B] Verbindungen der allgemeinen Formel (V)
A-NH-CO₂-X (V)
in welcher
A die oben angegebene Bedeutung hat
und
X für eine typische Schutzgruppe steht,
in inerten Lösemitteln und in Anwesenheit einer Base mit Epoxiden der allgemeinen Formel (IV) umsetzt,
oder
[C] im Fall R¹ = OH, zunächst Verbindungen der allgemeinen Formel (III) durch Abspaltung von Stickstoff in Alkoholen in die Verbindungen der allgemeinen Formel (Va)
A-NH-CO₂-Y (Va)
in welcher
A die oben angegebene Bedeutung hat
und
Y für geradkettiges oder verzweigtes C₁-C₆-Alkyl, vorzugsweise n-Butyl steht,
überführt,
und in einem zweiten Schritt wie unter [A] beschrieben in inerten Lösemitteln und in Anwesenheit einer Base mit Epoxiden der allgemeinen Formel (IV) umsetzt,
oder
[D] Verbindungen der allgemeinen Formel (V) zunächst durch Umsetzung mit Allylbromid in inerten Lösemitteln und in Anwesenheit einer Base in die Verbindungen der allgemeinen Formel (VI) 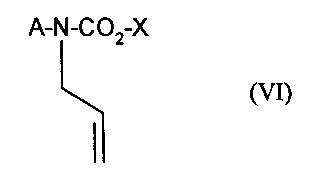 in welcher
A und X die oben angegebene Bedeutung haben,
überführt, anschließend mit Osmiumtetroxid / N-Methyl-morpholin-N-oxid die Verbindungen der allgemeinen Formel (VII) 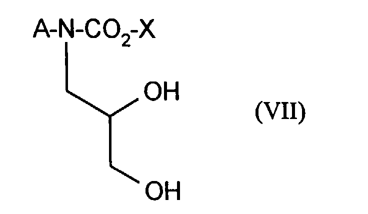 in welcher
A und X die oben angegebene Bedeutung haben,
herstellt und in einem letzten Schritt mit Basen in Acetonitril, vorzugsweise mit Kaliumcarbonat, eine Cyclisierung durchführt,
oder
[E] Verbindungen der allgemeinen Formel (VIII) 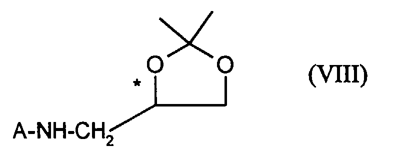 in welcher
A die oben angegebene Bedeutung hat,
entweder direkt mit Säuren und Kohlensäurediethylester
umsetzt,
oder zunächst durch Umsetzung der Verbindungen der allgemeinen Formel (VIII) mit Säuren die Verbindungen der allgemeinen Formel (IX) 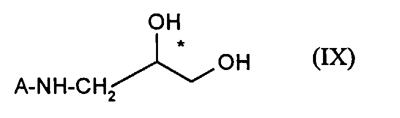 in welcher
A die oben angegebene Bedeutung hat,
herstellt,
und anschließend in Anwesenheit eines Hilfsmittels und/oder einer Säure in inerten Lösemitteln cyclisiert,
oder
[F] zunächst die heterocyclischen Amine (A-NH₂) mit einer Verbindung der allgemeinen Formel (IV) zu einer Verbindung der allgemeinen Formel (IX) umsetzt und anschließend mit Carbonyldiimidazol / Methylenchlorid, (EtO)₂CO oder Phosgen, Diphosgen oder Triphosgen wie unter [E] beschrieben cyclisiert,
oder
[G] zunächst Verbindungen der allgemeinen Formel (Ia) 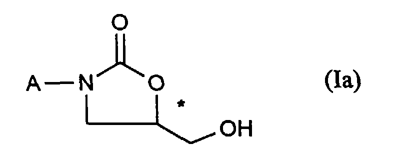 in welcher
A die oben angegebene Bedeutung hat,
durch Umsetzung mit (C₁-C₄)-Alkyl- oder Phenylsulfonsäurechloriden in inerten Lösemitteln und in Anwesenheit einer Base in die entsprechenden Verbindungen der allgemeinen Formel (Ib) 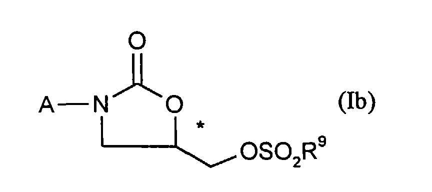 in welcher
A und R⁹ die oben bzw. in Anspruch 1 angegebene Bedeutung
haben,
überführt,
anschließend mit Natriumazid in inerten Lösemitteln die Azide der allgemeinen Formel (Ic) 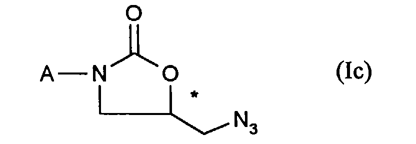 in welcher
A die obenangegebene Bedeutung hat,
herstellt,
in einem weiteren Schritt durch Umsetzung mit (C₁-C₄-O)₃-P oder PPh₃, in inerten Lösemitteln und mit Säuren in die Amine der allgemeinen Formel (Id) 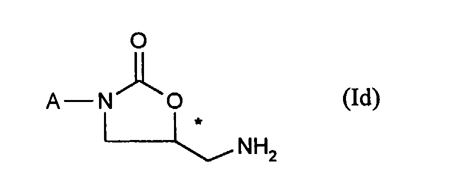 in welcher
A die oben angegebene Bedeutung hat,
überführt,
und durch Umsetzung mit Acetanhydrid oder anderen Acylierungsmitteln der allgemeinen Formel (X)
R¹⁸-CO-R¹² (X)
in welcher
R¹² die in Anspruch 1 angegebene Bedeutung hat
und
R¹⁸ für Halogen oder für den Rest -OCOR¹² steht,
in inerten Lösemitteln die Verbindungen der allgemeinen Formel (Ie) 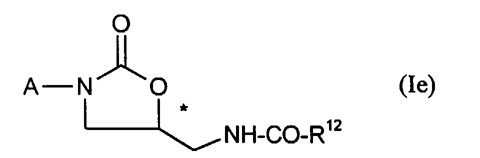 in welcher
A und R¹² die oben angegebene Bedeutung haben,
herstellt,
im Fall R¹ = NR¹⁰-CSR^{12'} Verbindungen der allgemeinen Formel (Id) mit Ethyldithiocarboxylaten und Triethylamin und im Fall R¹ = NR¹²-CS-NR¹⁶R¹⁷ mit Thioisocyanaten umsetzt,
die Verbindungen gegebenenfalls in ihre Salze überführt, und daß man gegebenenfalls in an sich bekannter Weise die Stereoisomeren trennt.

6. Verbindungen der allgemeinen Formel (I) gemäß Anspruch 1 zur Verwendung bei der Bekämpfung von Krankheiten.

7. Verwendung von Verbindungen der allgemeinen Formel (I) gemäß Anspruch 1 zur Herstellung von Arzneimitteln.

8. Arzneimittel enthaltend Verbindungen der allgemeinen Formel (I) gemäß Anspruch 1.
